# EUROPEAN PATENT APPLICATION

(11) **EP 4 461 803 A1**
(43) Date of publication of application: **13.11.2024**
(21) Application number: 23315187.7
(22) Date of filing: 11.05.2023
(51) Int. Cl.: C12M 3/00, C12M 1/00, C12M 1/42, C12M 1/34, C12M 1/36

(54) **MODULAR ELECTROPORATION METHOD AND SYSTEM**

(71) Applicant: Cellectis S.A., 75013 Paris (FR)
(72) Inventor: Lotteau, Luc, 78180 MONTIGNY-LE-BRETONNEUX (FR); Cazaux, Clément, 78180 MONTIGNY-LE-BRETONNEUX (FR); Celliere, Jean-Claude, 78180 MONTIGNY-LE-BRETONNEUX (FR); Sourdive, David, 75013 PARIS (FR); Cogoni, Sabrina, 75013 PARIS (FR); Derniame, Sophie, 75013 PARIS (FR)
(74) Representative: Santarelli

(57) **Abstract**

A device is provided for delivering electrical signals to an electroporation chamber and for monitoring the quality of the delivered signals. Specifically, the device is of a modular construction comprising a main module and an audit module. The main module has control electronics with a signal generator configured to generate electrical pulses for electroporation and is electrically connected to the audit module. The audit module is configured to store calibration data and to measure with respect to time current and voltage passed from the main module and to pass such measurement data to the main module. The main module is configured to generate an error signal if the voltage and current measured by the audit module does not conform with expected values of voltage and current. The audit module is removably attached to the main module.

## Description

### Field of the Invention

This invention relates to an electroporation machine. In particular, it relates to a modular electroporation system and to the method of operation of this system.

### Background

Electroporation is understood as the use of electrical pulses to reversibly destabilize biological membranes such as vesicle or cell membranes and during the period of destabilization to provide a pathway for the introduction of exogenous material into the vesicles or cells. Typically, such introduced exogenous material can be genetic material to obtain genetically modified cells used for research or therapeutic purposes. Both the cells and the exogenous material are supplied as a liquid suspension. Of considerable interest in recent years has been the use of electroporation to introduce mRNA into cells that are readily translated into proteins that confer additional functions to those cells. Cell therapy is an emergent area of medicine that requires sterile and reliable manufacturing facilities where primary cells originating from donors or patients can be genetically modified in large batches pursuant to strict good manufacturing practices (GMP). The production of clinical batches of allogeneic CAR T-cells, for instance, which requires several gene editing steps to ablate external receptors and express artificial chimeric antigen receptors (CAR) [Depil, S., et al. (2020) 'Off-the-shelf' allogeneic CAR T cells: development and challenges. Nat Rev Drug Discov 19: 185-199] is particularly challenging in that respect.

US 2006/0089674 discloses a system comprising a pulse voltage waveform generator, a switching device to connect the anode or cathode of the pulse voltage waveform generator to an electroporation chamber, and an electrode array within the electroporation chamber to convert the pulse voltage into a pulsed electric field within the chamber. Also disclosed is a cycle of filling of the electroporation chamber with a suspension of cells and exogenous material, electroporation of that mixture and emptying of the chamber in situations where the amount of cell suspension to be processed exceeds the capacity of the chamber.

During electroporation, in keeping with GMP and in order to ensure consistent quality of electroporation results, it is desirable to monitor and appraise the electrical pulses being applied to the electroporation chamber so as to assure among other things, quality control of the product. Such appraisal must take into consideration voltage and current as well as the timings of the pulses. By timing is to be understood both the duration of the pulses as well as the temporal spacing of successive pulses.

Typically, electroporation voltage and current data measured with respect to time is stored on a non-volatile memory for assessment and quality control at a later date.

As is known, however, the output of electronic circuitry is known to drift with time. Not only is this true of the circuitry, the signal generator, which is producing the electroporation pulses but also to that circuitry which monitors the voltage and current constituting those pulses. Traditionally, in order to assess, in particular, the self-monitoring capability of the electronic circuitry, outside calibration equipment has been connected to the electroporation system. This is, however, undesirable for a number of reasons. Specifically, electroporation equipment must be operated under cleanroom conditions thus the introduction of external equipment into proximity with the location where electroporation is being conducted or the removal of equipment for maintenance and calibration can lead to problems of contamination. Further, in view of this, it has been impossible to calibrate without moving/demobilizing the device. Moving and/or demobilizing the device leads to a loss of production with associated wasting of time and expense.

It is therefore the objective of the disclosed invention to present an electroporation system which is both inexpensive to construct and operate, which ensures good electroporation results, and which does not suffer from the drawbacks recited above.

Beyond the production of therapeutic grade cells, applications can be found in various area of research or of industrial processes such as the transformation of cells lines, bacteria, yeasts, algae, plant cells, protoplasts for bio-production, or the production of vaccines, as non-limiting examples.

### Definitions

Modular describes a construction based on modules which may or may not be integral. For the present invention, the modules comprise a main module and an audit module.

The system of the invention is designed to process vesicles, which can be artificial or living cells. The system has been particularly designed for the electroporation of cells, such as cell lines or primary cells.

Cell means a living cell that has been cultured or placed into suspension.

By "primary cell" or "primary cells" are intended cells taken from living tissue (e.g. biopsy material) and established for growth in vitro for a limited amount of time, meaning that they can undergo a limited number of population doublings. Primary cells are opposed to continuous tumorigenic or artificially immortalized cell lines. Non-limiting examples of such cell lines are CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells. Primary cells are typically used in cell therapy as they are deemed more functional and less tumorigenic.

Cells differentiated from stem cells, such as cord blood stem cells, progenitor cells, bone marrow stem cells, hematopoietic stem cells (HSC) and induced pluripotent stem cells (iPS) are considered as primary cells as per the present invention.

In general, primary cells originate directly or indirectly, preferably directly, from donors or patients through a variety of methods known in the art, as for instance by leukapheresis techniques as reviewed by Schwartz J.et al. [Guidelines on the use of therapeutic apheresis in clinical practice-evidence-based approach from the Writing Committee of the American Society for Apheresis: the sixth special issue (2013) J Clin Apher. 28(3):145-284].

Particular attention has been paid to immune cells in this invention, such as T-cells and NK cells, stem cells, such as Hematopoietic Stem and Progenitor Cells, Embryonic Stem (ES) cells or induced Pluripotent Stem (iPS) cells in view of producing clinical batches of therapeutic cells for cell therapy.

By Electroporation is meant the creation of transient permeability in cell membranes using pulsed electric fields without loss of cell viability.

Exogenous material is any substance outside living cells. For the purposes of this disclosure, it is material that is to be delivered into cells. A non-exclusive list of materials is polypeptides, polynucleotides, pharmaceuticals, polymers, carbohydrates and combinations of these in the same or different molecules. Examples of polypeptides and polynucleotides are proteins, and DNA or RNA (including mRNA, RNAi) respectively. Exogenous material for use in electroporation may include combinations of the above such as Ribonucleoproteins (protein and RNA complex).

PulseAgile protocol: A sequence of at least three waveforms that has one, two, or three of the following characteristics (1) at least two of the at least three waveforms differ from each other in waveform amplitude, (2) at least two of the at least three waveforms differ from each other in waveform width, and (3) a first waveform interval for a first set of two of the at least three waveforms is different from a second waveform interval for a second set of two of the at least three waveforms. Examples of such agile pulse sequences are described in patent US 6010613.

Suspension: Insoluble particles such as living cells, suspended in a water-based liquid.

### Summary of the Invention

The invention relates to an electroporation device, system and method.

In particular, the disclosure relates to a modular electroporation device comprising a main module and an audit module. The names "main" and "audit" are not intended to limit the disclosure but merely to describe the functionality of each module in general terms. The main module comprises control electronics with a signal generator configured to generate electrical pulses for electroporation, a multi-electrode connector connected to the control electronics and an output connector, for connection to an electroporation chamber. The audit module comprises a multi-electrode connector configured to mate with the multi-electrode connector of the main module. Further, the audit module is furnished with a non-volatile memory configured to store calibration data and sensor electronics connected to the multi-electrode connector and configured to measure a voltage and a current with respect to time of the electrical pulses received from the main module. The sensor electronics is further configured to transmit to the main module a digital communication based on the measured voltage and current and on the calibration data stored in the non-volatile memory.

The control electronics of the main module is configured to generate an error signal if the current and voltage measured by the sensor electronics and transmitted via the communication channel, having regard to the calibration data, does not match an expected current and voltage.

The audit module is removably attached to the main module. Optionally, the audit module is nested within a slot in the main module.

Optionally, the signal generator is configured to generate electrical pulses having a sequence of at least three waveforms comprising: (a) at least two of the at least three waveforms differing from each other in waveform amplitude; (b) at least two of the at least three waveforms differing from each other in waveform width, and (c) a first waveform interval for a first set of two of the at least three waveforms being different from a second waveform interval for a second set of two of the at least three waveforms.

Optionally, the audit module is configured to draw electrical power from the main module. Further, the sensor electronics may comprise an analogue to digital converter. Yet further, the sensor electronics may be configured to transmit to the main module the measured voltage and current upon receipt of a trigger signal from the main module.

A further aspect of the present disclosure is a system for electroporation comprising the electroporation device described above, an electroporation chamber and a calibration device configured to connect with the audit module when the audit module is separated from the main module. The calibration device comprises circuitry configured to calibrate the audit device and to store calibration data on the non-volatile memory.

The system disclosed above may also comprise a container for holding a suspension of cells, a container for holding a suspension of exogenous material, a container for receiving electroporation product and an arrangement of tubing configured to supply a predefined quantity of the suspension of cells and a predefined quantity of the suspension of exogenous material to the electroporation chamber (200) prior to electroporation and, following electroporation to deliver the product of electroporation from the electroporation chamber to the container for receiving electroporation product. The container for holding the suspension of cells representatively contains suspension of cell being of cell types selected from: CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells; further T cells, NK cells, cord blood stem cells, progenitor cells, bone marrow stem cells, hematopoietic stem cells (HSC) and induced pluripotent stem cells (iPS) and equivalent. The container for holding the suspension of exogenous material representatively contains a suspension of exogenous material selected from: polypeptides, polynucleotides including DNA and RNA, pharmaceuticals, polymers, carbohydrates and combinations of these including Ribonucleoproteins.

The disclosed system may further comprise a replacement audit module with the replacement audit module having the same functionality as the audit module.

In a further aspect, the disclosure also relates to a method of operating the electroporation system disclosed above. This method comprises: a) connecting the audit module to the calibration device; performing a calibration routine on the audit module; c) storing calibration information relating to the audit module on the non-volatile memory; d) disconnecting the audit module from the calibration device; e) connecting the audit module to the main module; f) energizing the main module to perform an electroporation sequence; g) measuring, with respect to time, an electroporation voltage and current by the audit module; h) correcting the measures voltage and current using calibration information stored on non-volatile memory; i) passing voltage and current information from the audit module to the main module; and j) comparing at the main module corrected voltage and current information with an expected condition. If the result of the comparison is outside the predefined tolerance, an error signal is generated.

Also disclosed are further steps to the method set out above wherein a second audit module is also calibrated following steps a) to d) above. The audit module is then connected to the main module and the electroporation steps e) to j) performed. Steps e) to j) can be repeated for a predefined time or number of cycles following which, the audit module is replaced with the second audit module. Steps e) to j) can then be performed using the combination of the main module with the second audit module.

The method set out above may also include the steps that prior to energizing the main module, the following is introduced into the electroporation chamber: a predefined quantity of a suspension of cells being of cell types selected from: CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells; further T cells, NK cells, cord blood stem cells, progenitor cells, bone marrow stem cells, hematopoietic stem cells (HSC) and induced pluripotent stem cells (iPS) and equivalent; and a predefined quantity of a suspension of exogenous material selected from: polypeptides, polynucleotides including DNA and RNA, pharmaceuticals, polymers, carbohydrates and combinations of these including Ribonucleoproteins.

Thanks to the configuration of the modular electroporation device and system described above, calibration can be maintained without having to interrupt the electroporation process for longer than necessary and without having to introduce extraneous equipment into the electroporation environment. Thanks to the claimed invention, it is possible to retain calibration of a continuously operating electroporation system whilst maintaining cleanroom conditions.

In the accompanying drawings, given by way of non-limiting examples:
Figure 1 is schematic diagram of a modular electroporation device;
Figures 2a, 2b and 2c are schematic diagrams showing three configurations of an electroporation system;

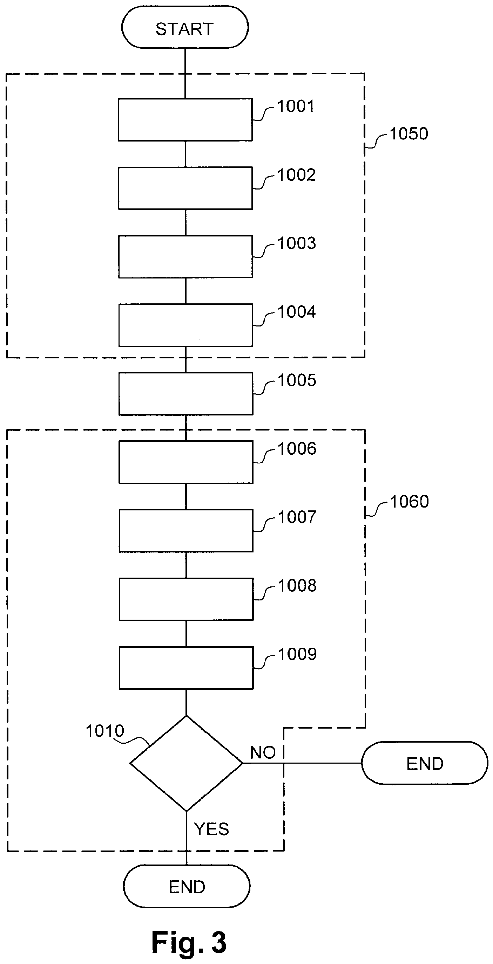

Figure 3 is a flowchart showing operation of the electroporation system of figs. 2a and 2b;
Figure 4 is a flowchart showing operation of the electroporation system of figs. 2a and 2c.
Figure 5 is an example of a modular electroporation device.

### Detailed Description

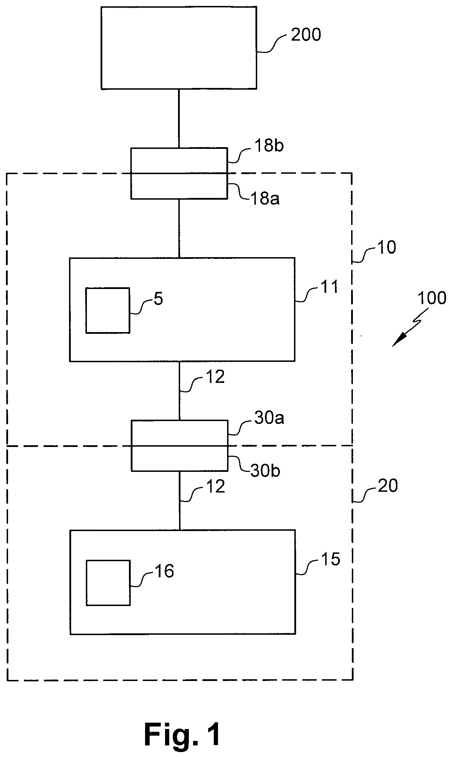

Fig. 1 shows a modular electroporation device 100 connected to an electroporation chamber 200. In the context of this disclosure, the phrase 'connected to an electroporation chamber' infers that an electrical connection is made to electrodes within an electroporation chamber such as to deliver an electric field to the contents of the chamber.

The electroporation device 100 comprises a main module 10 and detachable audit module 20. The main module 10 and the audit module 20 are connected by a multi-electrode connector 30a, 30b having a main side part 30a and an audit side part 30b. A suitable such multi-electrode connector 30a, 30b is a 31-way Harting connector.

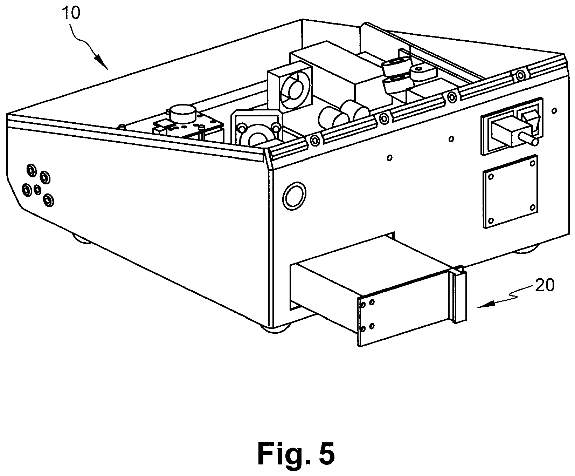

The main module 10 and the audit module 20 may also be further attached to one another mechanically. Further, the audit module 20 may be mechanically attached to the main module 10 by way of self-guiding rails and fixed to the rear panel by captive screws. In some arrangements, the audit module may by partially enclosed by the main module 10. For example, as shown in the example of Fig. 5, the audit module may fit into a slot in the main module.

Each of the main module 10 and the audit module 20 will now be described in turn. The main module comprises control electronics 11 comprising a signal generator 5 configured to generate electrical pulses for electroporation. The duration and voltage of the electrical pulses is chosen according to the biological material on which electroporation is being performed. Further, the shape of the voltage profile can also be controlled. An example signal generator is configured to generate signals conforming to the PulseAgile protocol.

An output electrical connector 18a, 18b is configured to connect the main module 10 to the electroporation chamber 200. In some arrangements this electrical connector 18a, 18b is separable. Signals generated by the signal generator 5 are passed through the output electrical connector to the electroporation chamber 200.

The audit module 20 contains sensor electronics 15 which are connected to the multi-electrode connector. The sensor electronics also comprise an analogue to digital converter and a non-volatile memory 16.

The electrical connection 12 between the main module and the audit module passes through the multi-electrode connector. The electrical connection comprises a number of components including: wiring carrying the electroporation signals generated by the signal generator 15; and a digital communication channel configured for communication between the audit module 20 and the main module 10. The digital communication channel may be for example a serial bus such as an RS232. The electrical connection 12 may also be configured to supply electrical power from the main module to the audit module. In some other arrangements, the audit module comprises its own power supply.

The sensor electronics 15 are configured to measure the voltage and/or current of the electroporation signals generated by the signal generator and conveyed to the audit module through the electrical connection 12. The voltage and/or current are recorded with respect to time. Preferably, the electroporation signals are routed through the audit module. The sensor electronics 15 of the audit module 20 are configured to measure a current and a voltage of the electroporation signals as they pass through the audit module. That is to say, the electroporation signals are generated in the main module by the signal generator 5; the signals are then routed through the audit module; back to the main module and then to the electroporation chamber 200.

As set out above, a digital communication channel is provided between the audit module 20 and the main module 10. Further, the sensor electronics are configured to represent the current and voltage measured with respect to time and to pass this information digitally through the digital communication channel to the control electronics of the main module.

Further as set out above, the audit module comprises a non-volatile memory 16. Specifically, the non-volatile memory is configured to store calibration information pertaining to the audit module's sensitivity to the measured current and voltage. Accordingly, the measurements of current and voltage are corrected according to the calibration data. Preferably, such correction is performed by the sensor electronics 15 prior to communication of corrected voltage and current information through the digital communication channel to the main module 10. Alternatively, uncorrected current and voltage information as well as calibration information is passed through the digital communication channel for the voltage and current information to be corrected at the control electronics 11 of the main module 10.

in some arrangements, the audit module is configured to continuously measure the current and voltage and to pass this information via the digital communication channel to the main module. Alternatively, measurement and passing of information is commenced upon receipt of a trigger signal from the main module. Upon receipt of this trigger signal the sensor electronics is configured to measure the current and voltage of the electroporation signal and to communicate this information digitally to the main module 10. Alternatively, the audit module is configured to pass, upon receipt of a trigger signal, previously measured values of current and voltage measured with respect to time.

As illustrated, the audit module 20 draws its electrical power from the main module 10 although in other arrangements, the audit module has its own source of power.

A time based log of voltage and current as measure by the audit module is stored on a non-volatile memory. This non-volatile memory may constitute a part of the main module 10 or of the audit module 20.

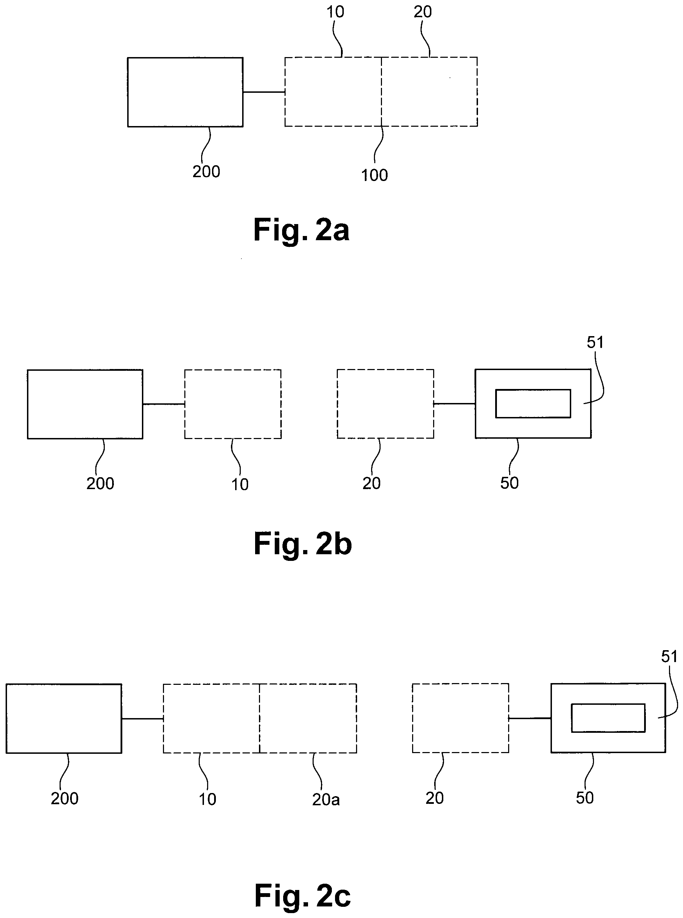

With reference to fig. 2a - 2c it will now be described how the modular electroporation device 100 described above is incorporated into an electroporation system. As depicted in fig. 2a, the modular electroporation device 100 described above is connected to an electroporation chamber 200. During electroporation, the voltage and current of the electroporation signals is measured by the sensor electronics 15 of the audit module and this information is passed to the control electronics 11 of the main module 10 via the digital communication channel. The measured voltage and current is corrected using calibration information stored in the non-volatile memory 16 of the audit module 20. At the main module, the voltage and current information is compared with an expected value. This comparing may include measured verses expected temporal qualities of the electrical signals comprising pulse width and time between successive pulses. On the basis of this comparison, the control electronics is configured to generate an error signal if the measured values are not within predefined tolerances of the expected values. Where such an error signal has been generated, the resulting electroporation product cannot be used and must be rejected. Where no error signal has been generated, the electroporation product can be accepted for its intended purpose.

Fig. 2b depicts how the audit module 20 is not connected to the main module 10 and has been instead connected to a calibration device 50. The calibration device 50 contains circuitry 51 configured assess the current and voltage measuring capabilities of the audit module and to write calibration information onto the non-volatile memory 16. This calibration information is used, as described above, to correct voltage and current measurements performed by the audit module.

In Fig. 2c is shown a further arrangement wherein a replacement audit module 20a is attached to the main module 10. This can occur at the same time that the audit module 20 is attached to the calibration device 50.

Typically, the electroporation chamber 200 will be fluidly connected to an arrangement of tubing configured to supply a predefined quantity of a suspension of cells from a container holding a suspension of cells and a predefined quantity of a suspension of exogenous material from a container holding a suspension of exogenous material. Following electroporation the arrangement of tubing is further configured to deliver the product of electroporation from the electroporation chamber (200) to a container for receiving electroporation product.

The container for holding the suspension of cells representatively contains suspension of cell being of cell types selected from: CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells; further T cells, NK cells, cord blood stem cells, progenitor cells, bone marrow stem cells, hematopoietic stem cells (HSC) and induced pluripotent stem cells (iPS) and equivalent.

The container for holding the suspension of exogenous material representatively contains a suspension of exogenous material selected from: polypeptides, polynucleotides including DNA and RNA, pharmaceuticals, polymers, carbohydrates and combinations of these including Ribonucleoproteins.

It will now be described the method 1000 for operating the electroporation system depicted in figs. 2a - 2b above.

In a connecting step 1001, the audit module 20 is connected to the calibration device 50. Thereafter, the calibration device is energized such as to perform 1002 a calibration routine on the audit module. For example, during this step, the calibration device supplies voltages and currents of a known magnitude to the audit device and the output of the audit device assessed by the calibration device. On the basis of this, the calibration device determines calibration information necessary to correct any error in the measurements performed by the audit device 20.

The calibration information thus determined by the calibration device 50 is then stored on the non-volatile memory 16 of the audit device. Following this step, the audit module is disconnected 1004 from the calibration device.

Collectively, steps 1001, 1002, 1003 and 1004 can be regarded as calibration steps 1050 equipping the non-volatile memory with the necessary calibration information. In some instances, the first such calibration is performed during the manufacturing process.

Subsequently, in a connecting step 1005, the audit module is connected to the main module 10. The main module is then energized 1006 so as to perform an electroporation sequence. As constituent part of this electroporation sequence, electroporation signals are generated by the main module which are applied to the electroporation chamber 200. During this electroporation sequence, the voltage and current being supplied to the electroporation chamber 200 is measured by the audit module 20.

Following measurement, voltage and current measurements are corrected 1008 on the basis of the calibration information stored in the non-volatile memory 16 and passed 1009 by way of the communication channel from the audit module 20 to main module 10. Preferably, the calibration is performed at the audit module prior to the voltage and current information being communicated to the main module 10. Alternatively, raw voltage and current data may be communicated from the audit module 20 to the main module 10 together with the calibration information and correcting of the voltage and current information performed, on the basis of the calibration data, at the main module 10. In preferred arrangements a timestamp is affixed to each current and voltage measurement. Data is communicated from the audit module to the main module either as it is measured and/or upon receipt of a trigger signal by the audit module from the main module.

At step 1010, at the main module, the corrected voltage and current information is compared with expected values. The comparing relates to both the magnitude of the current and voltage as well as temporal aspect of pulse width and distance between pulses. If the result of this comparison is outside the predefined tolerance, an error signal is generated by the main module. In this case, the results of the electroporation sequence do not meet the requirements of GMP so stand to be rejected. Following such a rejection, the electroporation device must, for example, be sent for service.

Collectively, steps 1006, 1007, 1008, 1009 and 1010 can be regarded as the electroporation steps 1060 during which electroporation occurs and the electroporation signals are audited and compared with the expectation with an error signal being generated as appropriate.

As is appropriate for this method, prior to energizing 1006 the main module 10, the material to be electroporated is introduced into the electroporation chamber 200. Representatively, this consists of a predefined quantity of a suspension of cells being of cell types selected from: CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells; further T cells, NK cells, cord blood stem cells, progenitor cells, bone marrow stem cells, hematopoietic stem cells (HSC) and induced pluripotent stem cells (iPS),
and a predefined quantity of a suspension of exogenous material selected from: polypeptides, polynucleotides including DNA and RNA, pharmaceuticals, polymers, carbohydrates and combinations of these including Ribonucleoproteins, and combinations of these.

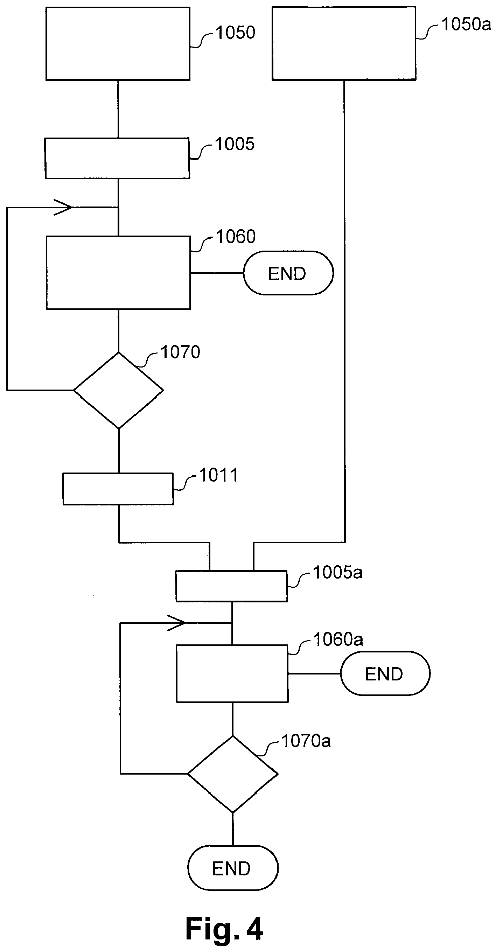

Fig. 4 shows a method (1000) of operating the electroporation system described above and further incorporating the arrangement shown in fig. 2c. That is to say, the use of the second audit module 20a is provided. Here, the method is the same as provided above in that the calibration steps 1050 are performed on the audit module. Further, the second audit module 20a is also calibrated by way of an analogous method to that of step 1050a set out above in relation to the audit module 20. The second audit module 20a can be calibrated while the audit module 20 is connected to the main module or at any other time consistent with the second audit module 20a being ready for use when required.

The method proceeds by performing step 1005 of connecting the audit module 20 to the main module followed by the electroporation steps 1060 using the audit module to audit the electroporation signals supplied to the electroporation chamber 200 and to reject the electroporation sequence where the voltage and current are not within the allowed tolerances such that an error signal has been generated.

Assuming that no such rejection has occurred, the voltage and current are determined as being within the allowed tolerances, the electroporation steps 1060 can be repeated either for a predetermined amount of time, until a predetermined number of electroporation cycles has been reached or until the logic at step 1010 returns an error. Decision box 1070 determines whether the cycle is to be repeated based on these considerations.

Upon reaching a predetermined time or number cycles the audit module 20 is disconnected 1011 from the main module 10 and substituted 1005a for the second audit module 20a. The electroporation steps 1060a are then performed using the combination of the main module 10 and the second audit module 20a. The audit module, thus released from service, can now be calibrated without the need to halt operation of the electroporation device which continues to run thanks to the combination of the main module with the second audit module 20a. Electroporation cycles are continued with this combination until, as with the main module 10 audit module 20 combination, a predetermined time or number cycles is attained 1070a.

Although not depicted here, it will be appreciated that while the second audit module 20a is connected to the main module 10, a calibration can be performed on the audit module 20. Likewise, further audit modules can be calibrated and swapped in as necessary. In this way, the amount of downtime of the electroporation device can be kept to a minimum whilst ensuring consistency in quality of the results.

Fig. 5 shows a representative example of a system having a main module 10 into which is connected an audit module 20 via a multi-pin connector. In this representation, the audit module is positioned within a shallow recess within the main module. It is appreciated that the audit module can be easily removed for calibration or replacement.

The reader will appreciate the above described electroporation device, system and method of operation represents a welcome improvement in the cell industry. The disclosed device system and method allows continued high quality calibration and thus electroporation results over extended production runs without the need to withdraw electroporation equipment for calibration.

## Claims

1. A modular electroporation device (100) comprising:
a main module (10) comprising:
control electronics comprising a signal generator (5) configured to generate electrical pulses for electroporation;
a multi-electrode connector (30a) connected to the control electronics comprising: at least one electrical conduit in electrical connection with the signal generator; and a digital communication channel;
an output connector (18a, 18b), for connection to an electroporation chamber to pass the electrical pulses generated by the signal generator;
an audit module (20) comprising:
a multi-electrode connector (30b) configured to mate with the multi-electrode connector (30a) of the main module (10) so as to: receive the electrical pulses generated by the signal generator; and to carry digital communication between the audit module (20) and the main module (10) via the digital communication channel;
a non-volatile memory (16) configured to store calibration data;
sensor electronics (15) connected to the multi-electrode connector (30b) and configured to measure, with respect to time, a voltage and a current of the electrical pulses received from the main module (10);
where the sensor electronics (15) is further configured to transmit a communication via the digital communication channel based on the measured voltage and current and on the calibration data stored in the non-volatile memory (16);
wherein the control electronics is configured to generate an error signal if current and voltage with respect to time measured by the sensor electronics and transmitted via the communication channel, having regard to the calibration data, does not match an expected current and voltage with respect to time;
**characterized in that** the audit module (20) is removably attached to the main module (10).

2. The modular electroporation device (100) according to claim 1 wherein the error signal is generated if the time between consecutive electrical pulses does not match an expected time or if the temporal width of the electrical pulses does not match an expected temporal width.

3. The modular electroporation device (100) according to claim 1 or 2 wherein the audit module 20 is nested within a slot in the main module 10.

4. The modular electroporation device (100) according to any of claims 1 to 3 wherein the signal generator (5) is configured to generate electrical pulses having a sequence of at least three waveforms comprising:
(a) at least two of the at least three waveforms differing from each other in waveform amplitude,
(b) at least two of the at least three waveforms differing from each other in waveform width, and
(c) a first waveform interval for a first set of two of the at least three waveforms being different from a second waveform interval for a second set of two of the at least three waveforms.

5. The modular electroporation device of any of claims 1 to 4 wherein the audit module (20) is configured to draw electrical power from the main module (10).

6. The modular electroporation device of any of claims 1 to 5 wherein the audit module or the main module comprises an analogue to digital converter and is further configured to store on a non-volatile memory, a record of the current and voltage with respect to time of the electrical pulses as measured by the audit module and transmitted to the main module.

7. The modular electroporation device of any of claims 1 to 6 wherein:
the main module (10) is configured to generate a signal trigger;
the audit module (20) is configured to receive the signal trigger; and
the sensor electronics (15) are configured transmit the communication of the measured voltage and current upon receipt of the signal trigger.

8. A system for electroporation comprising:
the electroporation device (100) of claims 1 to 7;
an electroporation chamber (200);
a calibration device (50) configured to connect with the audit module (20) when the audit module is separated from the main module (10);
the calibration device (50) comprising circuitry (51) configured to calibrate the audit device (20) and to store calibration data on the non-volatile memory (16).

9. The system of claim 8 further comprising:
a container for holding a suspension of cells;
a container for holding a suspension of exogenous material;
a container for receiving electroporation product;
an arrangement of tubing configured to supply a predefined quantity of the suspension of cells and a predefined quantity of the suspension of exogenous material to the electroporation chamber (200) prior to electroporation and, following electroporation to deliver the product of electroporation from the electroporation chamber to the container for receiving electroporation product.

10. The system of claim 9 wherein:
the container for holding a suspension of cells contains suspension of cells being of cell types selected from: CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells; further T cells, NK cells, cord blood stem cells, progenitor cells, bone marrow stem cells, hematopoietic stem cells (HSC) and induced pluripotent stem cells (iPS);
the container for holding a suspension of exogenous material contains a suspension of exogenous material selected from: polypeptides, polynucleotides including DNA and RNA, pharmaceuticals, polymers, carbohydrates and combinations of these including Ribonucleoproteins.

11. The system of claim 8 to 10 further comprising a replacement audit module (20a), the replacement audit module (20a) having the same functionality as the audit module (20).

12. A method (1000) of operating the electroporation system of claim 8 to 10 comprising:
a) connecting (1001) the audit module to the calibration device;
b) performing (1002) a calibration routine on the audit module;
c) storing (1003) calibration information relating to the audit module on the non-volatile memory (16);
d) disconnecting (1004) the audit module (20) from the calibration device (50);
e) connecting (1005) the audit module (20) to the main module (10);
f) energizing (1006) the main module (10) to perform an electroporation sequence;
g) measuring (1007) by the audit module an electroporation voltage and current with respect to time;
h) correcting (1008) the measured voltage and current using calibration information stored on the non-volatile memory;
i) passing (1009) the voltage and current information from the audit module (20) to the main module (10);
j) comparing (1010) at the main module corrected voltage and current information with an expectation value wherein: if result of the comparison is outside the predefined tolerance generating an error signal.

13. A method (1000) of operating the electroporation system of claim 11 comprising operating the electroporation system according to claim 11 with the audit module; replacing the audit module (20) with the replacement audit module; operating the electroporation system with the replacement audit module.

14. The method of claim 12 or 13 wherein steps f) to j) are repeated until one of a predetermined time or a predetermined number of cycles has been reached.

15. The method of any of claims 12 to 14 further comprising, prior to energizing the main module (10), introducing into the electroporation chamber (200):
a predefined quantity of a suspension of cells being selected from: CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells; further T cells, NK cells, cord blood stem cells, progenitor cells, bone marrow stem cells, hematopoietic stem cells (HSC) and induced pluripotent stem cells (iPS) and equivalent;
a predefined quantity of a suspension of exogenous material selected from: polypeptides, polynucleotides including DNA and RNA), pharmaceuticals, polymers, carbohydrates and combinations of these including Ribonucleoproteins.
